(19) Europäisches Patentamt
European Patent Office
Office européen des brevets

(11) Numéro de publication: **0 192 522**
**B1**

(12) # FASCICULE DE BREVET EUROPÉEN

(45) Date de publication du fascicule du brevet: **31.08.88**

(51) Int. Cl.⁴: **C 07 D 211/42,** A 61 K 31/445

(21) Numéro de dépôt: **86400154.0**

(22) Date de dépôt: **27.01.86**

(54) **1-ethyl - 3-hydroxy-3-phénylpipéridine, procédé de préparation et utilisation en thérapeutique.**

(30) Priorité: **01.02.85 FR 8501410**
**10.07.85 FR 8510550**

(43) Date de publication de la demande:
**27.08.86 Bulletin 86/35**

(45) Mention de la délivrance du brevet:
**31.08.88 Bulletin 88/35**

(84) Etats contractants désignés:
**AT BE CH DE FR GB IT LI LU NL SE**

(56) Documents cité:
**EUROPEAN JOURNAL OF MEDICINAL CHEMISTRY, vol. 16, no. 2, mars-avril 1981, pages 163-169, Châtenay-Malabry, FR; A. BALSAMO et al.: "An approach to the knowledge of the steric requirements for alpha-adrenoreceptor direct activation. Alpha-Adrenergic properties of new 3-piperidinols"**

**Le dossier contient des informations techniques présentées postérieurement au dépôt de la demande et ne figurant pas dans le présent fascicule.**

(73) Titulaire: **LABORATOIRE L. LAFON Société anonyme dite:, 1 rue Georges Médéric, F-94701 Maisons- Alfort (FR)**

(72) Inventeur: **Lafon, Louis, 5 rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

**EP 0 192 522 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention concerne en tant que produits industriels nouveaux la 1-éthyl-3-hydroxy-3-phénylpipéridine de formule I ci-après et ses sels d'addition. Elle concerne également le procédé de préparation de la 1-éthyl-3-hydroxy-3-phénylpipéridine qui est utile en tant que (i) intermédiaire de syntnèse, et (ii) substance active en thérapeutique en égard notamment à ses propriétés anti-dépressives du système nerveux central (SNC).

On sait que l'on a déjà décrit dans le passé des dérivés de 3-hydroxy-3-phénylpipéridine dans lesquels le noyau phényle est toujours substitué en particulier par un ou plusieurs atomes d'halogène, groupes alkyle, groupes alkoxy, lesdits dérivés étant préconisés en tant que produits intermédiaires de synthèse.

Ainsi le document FR-A-2 496 099 décrit en particulier les 3-hydroxy-3-(3-méthoxyphényl)-pipéridine et 3-hydroxy-3-(3,4-diméthoxyphényl)-pipéridine comme intermédiaires dans la préparation de 1-alkyl-3-(phényl sub-stitué)-1,2,5,6-tétrahydropyridines; et, le document US-A-4 263 438 cite d'autres dérivés de 3-hydroxy-3-phénylpipéridine dans lesquels le noyau phényle est disubstitué (un groupe OH, alkoxy ou phénoxy en position ortho, et un groupe alkylèneoxyalkyle en position para).

Par ailleurs, FR-A-1 455 825 mentionne comme composés intermédiaires des 1-alkyl-4-(halogénopényl)-4-hydroxypipéridines.

On sait par ailleurs que l'article de A. BALSAMO et al., Eur. J. Med. Chem. - Chimica Therapeutica, 16 (N° 2), pages 163 - 169, (1981), décrit en tant que produits intermédiaires de synthèse un homologue inférieur et un homologue supérieur du composé de formule I ci-après suivant l'invention, à savoir les 1-méthyl et 1-isopropyl-3-hydroxy-3-phénylpiperidines, sans mention de leurs propriétés pharmacologiques potentielles.

Le composé de formule I suivant l'invention et ses sels d'addition se distinguent de l'enseignement des documents FR-A-2 496 099, US-A-4 263 438 et FR-A-1 455 825 précités par le fait que leur noyau phényle n'est pas polysubstitué, c'est-à-dire qu'il ne comporte aucun autre radical en plus du groupe pipéridinyle. Ils se distinguent des homologues inférieur et supérieur décrits par A. BALSAMO et al. précité par le choix du groupe alkyle substitué sur l'atome d'azote du cycle pipéridinyle, à savoir ici le groupe éthyle. De plus le composé de formule I et ses sels selon l'invention offrent l'avantage d'être dépourvus d'effets tératogènes néfastes à la différence de certains autres dérivés de 3-hydroxy-3-phénylpipéridine.

Les nouveaux dérivés selon l'invention sont caractérisés en ce qu'ils sont choisis parmi l'ensemble constitué par:

a) la 1-éthyl-3-hydroxy-3-phénylpipéridine de formule I

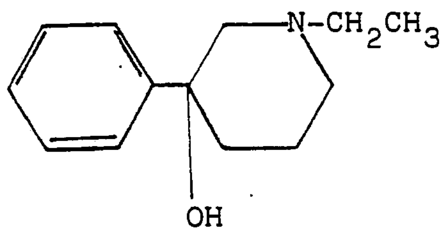

(I)

et,

b) ses sels d'addition.

Par sels d'addition, on entend ici, d'une part, les sels d'addition d'acide obtenus par réaction de la base libre de formule I avec les acides minéraux et organiques et, d'autre part, les sels d'ammonium. Parmi les acides utilisables pour salifier la base de formule I, on peut notamment mentionner l'es acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut également citer $ICH_3$ et $ClCH_3$. Les sels d'addition d'aacide sont les sels préférés d'un point de vue thérapeutique.

Un certain nombre de composés typiques selon l'invention a été consigné de façon nullement limitative dans le tableau I ci-après.

**TABLEAU I**

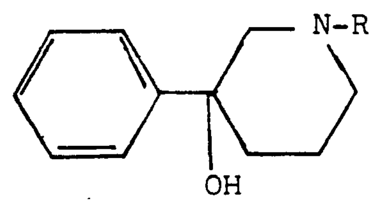

| Produit | No. de Code | R | $F_{(c)}$ |
|---|---|---|---|
| Ex. 1(a) | CRL 41 098 | $CH_2CH_3$ | 161°C |
| Ex. 2(b) | - | $CH_2CH_3$ | - |

Notes
(a) : chlorhydrate
(b) : méthanesulfonate
(c) : point de fusion instantanée

Le composé de formule I peut être préparé selon une méthode connue en soi par application de mécanismes réactionnels classiques. Le procédé de préparation que l'on préconise consiste à faire réagir un organomagnésien, à savoir un halogénure de phénylmagnésium de formule II

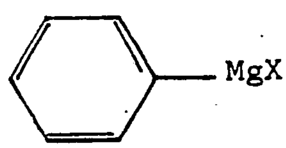

(II)

(où X est un atome d'halogène, notamment Cl, Br ou I, et de préférence Br) avec l'éthyl-3-pipéridinone de formule III

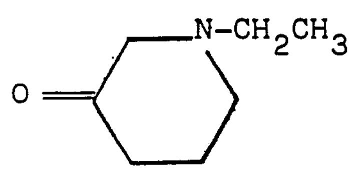

(III)

pendant au moins 0,25 h à une température comprise entre 0 et 25°C, sous atmosphère d'azote, dans un solvant inerte notamment choisi parmi les éthers (en particulier le diméthyléther, le diéthyléther, le diisopropyléther), le tétrahydrofuranne et leurs mélanges.

En pratique, on introduit l'éthyl-3-péridinone, préalablement dissoute dans un solvant inerte, dans une solution de l'halogénure de phénylmagnésium dans un solvant inerte identique ou différent du précédent, à une température de 0°C, que l'on maintient pendant la durée d'introduction de ladite éthyl-pipéridinone, puis on laisse après ladite introduction le milieu réactionnel revenir à la température ambiante (15 - 20°C). On opèrera en général avec un excès de $C_6H_5MgX$ par rapport aux conditions stoechiométriques, en particulier 2 à 3 moles de II pour 1 mole de III.

En variante, le composé deformule I peut également être préparé selon la réaction

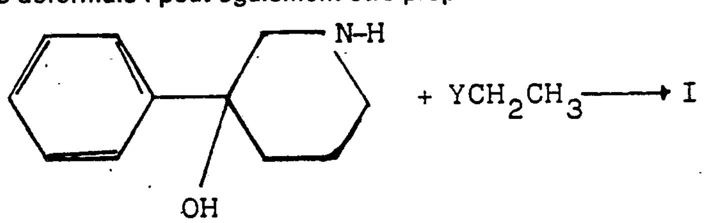


(VI)

où Y est un atome d'halogène (notamment F, Cl, Br ou, de préférence, I) à la température de reflux du milieu réactionnel, pendant au moins 2 h, à raison de 2 à 3 moles de $YCH_2CH_3$ pour 1 mole de VI.

Le composé de formule 1 et ses sels d'addition sont utiles en tant qu'agents antidépresseurs du SNC et présentent l'avantage d'être moins toxiques que les dérivés de N-alkyl-3-phényl-1,2,5,6- et 1,4,5,6-tétrahydropyridine correspondants de formules IV et V

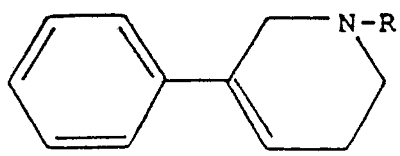

et

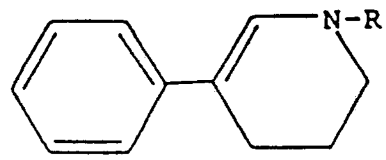

(IV) (V)

(où R est un groupe alkyle en $C_2$-$C_4$) décrits dans la demande de brevet français No. 8 501 410 du 1er février 1985, qui sont actifs sur le SNC en tant que sédatifs et qui peuvent être préparés à partir du composé de formule I et de ses homologues N-alkylés par déshydratation.

Selon l'invention on préconise une composition thérapeutique qui est caractérisée en ce qu'elle renferme, en association avec un excipient physiologiquement acceptable, la 1-éthyl-3-hydroxy-3-phénylpipéridine ou l'un de ses sels d'addition non-toxiques.

Bien entendu dans une telle composition, l'ingrédient actif, qui est choisi parmi l'ensemble comprenant la 1-éthyl-3-hydroxy-3-phénylpipéridine et ses sels d'addition, intervient en quantité pharmaceutiquement efficace.

On préconise par ailleurs l'utilisation du composé de formule I et de ses sels d'addition non-toxiques pour l'obtention d'un médicament anti-dépresseur du SNC destiné à une utilisation en thérapeutique humaine vis-à-vis des dépressions et des états dépressifs.

D'autres avantages et caractéristiques de l'invention seront mieux compris à la lecture qui va suivre (i) d'un exemple de preparation et (ii) de résultats d'essais neuropsychopharmacologiques. Ces éléments, nulement limitatifs, sont donnés à titre d'illustration.

PREPARATION I

Obtention du chlorhydrate de 1-éthyl-3-hydroxy-3-phényl-pipéridine.

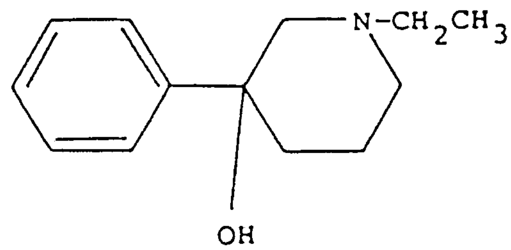

, HCl

(Exemple 1, No. de Code : CRL 41 098).

Autre nomenclature : chlorhydrate de N-éthyl-3-phényl-3-pipéridinol.

On coule 62 ml d'une solution 3 M de bromure de phénylmagnésium dans le diéthyléther, sous atmosphère d'azote, dans une solution refroidie (par un bain glace + sel) de 0,153 mole de 1-éthyl-3-pipéridinone dans le diéthyléther. L'introduction achevée, on laisse le milieu réactionnel revenir à température ambiante. On coule le milieu réactionnel dans la glace, décante la phase éthérée, lave la phase éthérée avec de l'eau, puis sèche sur $MgSO_4$. On filtre, puis à partir du filtrat, on précipite le chlorhydrate au moyen d'éthanol chlorhydrique. Par recristallisation du mélange acétone-éthanol (1:1) v/v on obtient 107 g (rendement 2,7 %) de CRL 41 098.

$F_{inst.}$ = 161 °C.

Analyse
- % $Cl^{\ominus}$ mesuré : 14,88 %
- % $Cl^{\ominus}$ théorique 14,70 %.

On a résumé ci-après les résultats des essais toxicologiques et neuropsychopharmacologiques qui ont été entrepris avec le produit préféré selon l'invention, à savoir le CRL 41 098 (Exemple 1). Dans ces essais ledit CRL 41 098 a été administré par voie intrapéritonéale, en solution à pH = 5 dans de l'eau distillée, sous un volume de 20 ml/kg chez la souris mâle.

I. TOXICITE

Chez la souris mâle par voie I. P. on constate que le CRL 41 098 ne provoque pas la mort aux doses de 16, 32, 64 et 128 mg/kg. La DL-O (dose maximale non mortelle) est donc supérieure à 128 mg/kg. La DL-50 du CRL 41 098 est de l'ordre d'environ 250 mg/kg.

Par comparaison chez la souris mâle par voie I. P. (i) les DL-O et DL-50 du CRL 41 244 [chlorhydrate de N-isopropyl-3-phényl-1,2,5,6- (ou 1,4,5,6-) tétrahydropyridine] sont respectivement de l'ordre de 30 mg/kg et de 90 mg/kg, et (ii) la DL-30 du CRL 41 124 [chlorhydrate de N-éthyl-3-phényl-1,2,5,6- (ou 1,4,5,6-) tétrahydropyridine] est de l'ordre de 60 mg/kg.

II. COMPORTEMENT GLOBAL ET REACTIVITES

Des lots de 6 animaux sont observés avant, puis 0,25 h, 0,50 h, 1 h, 2 h, 3 h et 24 h après l'administration de CRL 41 098. On constate chez la souris

aux doses de 1 et 4 mg/kg :

- un comportement, des réactivités, des variations de la température rectale et du diamètre pupillaire comparables aux animaux du lot témoin ne recevant que de l'eau distillée;

à la dose de 16 mg/kg:

- une sédation 0,50 h après administration, et
- une hypothermie (maximum de -2,5° C 0,50 h après administration) durant pendant 1 - 3 h et,

à la dose de 64 mg/kg

- une sédation pendant 0,50 h environ,
- une hypothermie (maximum de 2,7° C 0,50 h après administration) durant pendant 1,5 - 3 h.

III. ACTION SUR LA TEMPERATURE RECTALE

La température rectale des souris (6 animaux par dose) est notée toutes les 30 minutes pendant 3 h, après l'administration du CRL 41 098. On constate que, aux doses de 16 mg/kg et surtout 64 mg/kg, le CRL 41 098 provoque une hypothermie d'une durée de 1 h (dose de 16 mg/kg) à 1,5 h (dose de 64 mg/kg).

IV. INTERACTION AVEC L'APOMORPHINE

Le CRL 41 098 est administré à des lots de 6 souris 30 minutes avant l'injection sous-cutanée de 1 ou 16 mg/kg d'apomorphine. On observe que, à la dose de 64 mg/kg, le CRL 41 098 antagonise l'hypothermie induite par l'apomorphine, sans modifier l'attitude de verticalisation et les stéréotypes.

V. INTERACTION AVEC LA RESERPINE

Quatre heures après l'injection intrapéritonéale de 2,5 mg/kg de réserpine, des lots de 6 souris reçoivent le CRL 41 098. On note que le CRL 41 098 s'oppose à l'hypothermie réserpinique. Cependant l'antagonisme n'apparaît nettement qu'aux doses de 16 à 64 mg/kg. Par ailleurs, le ptôsis réserpinique n'est pratiquement pas modifié.

VI. INTERACTION AVEC L'OXOTREMORINE

Le CRL 41 098 est administré à des lots de 6 souris 0,5 h avant l'injection intrapéritonéale de 0,5 mg/kg d'oxotrémorine.

1) Action sur la température

On constate que le CRL 41 098 antagonise aux doses de 4 mg/kg, 16 mg/kg et 64 mg/kg l'effet hypothermisant de l'oxotrémorine.

2) Action sur les tremblements

On constate que le CRL 41 098 n'a pratiquement pas d'effets sur les tremblements induits par l'oxotrémorine.

3) Action sur les symptômes cholinergiques périphériques

On observe que le CRL 41 098 ne modifie pas les signes de stimulation cholinergique périphérique induits par l'oxotrémorine.

VII. ACTION SUR LA MOTILITE SPONTANEE

0,5 h après avoir reçu le CRL 41 098, les souris (6 par dose, 12 témoins) sont placées en actimètre où leur motilité est enregistrée pendant 30 minutes.

On constate que, aux doses de 16 et surtout de 64 mg/kg, le CRL 41 098 diminue nettement l'activité motrice spontanée de la souris.

L'ensemble de ces résultats met clairement en évidence l'intérêt du C-41 098 en tant qu'agent anti-dépresseur. Dans le profil neuropsychopharmacologique de ce produit on note également des effets sédatifs qui se manifestent à côté des propriétés essentielles anti-dépressives.

L'étude tératogène menée chez la lapine (groupe de 10 femelles par dose, et lot de 15 femelles pour contrôle) selon un protocole comprenant:

- l'administration de CRL 41 098 par gastrogavage à des doses quotidiennes de 0 mg/kg, 10 mg/kg, 50 mg/kg et 100 mg/kg, du 5è jour au 18è jour de la gestation, puis
- la mise en oeuvre d'une césarienne le 28è jour de la gestation,

a montré que, à la différence d'autres 3-hydroxy-3-phényl-pipéridines de l'art antérieur, le CRL 41 098 est dépourvu d'effet tératogène.

**Revendications** pour les états contractants: BE, CH,

DE, FR, GB, IT, LI, LU, NL, SE

1. Dérivé de 3-hydroxy-3-phénylpipéridine, caractérisé en ce qu'il est choisi parmi l'ensemble constitué par:
a) la 1-éthyl-3-hydroxy-3-phénylpipéridine de formule I

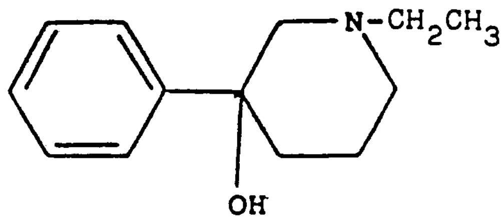

(I)

et
b) ses sels d'addition.

2. Chlorhydrate de 1-éthyl-3-hydroxy-3-phényl-pipéridine.

3. Composition thérapeutique, caractérisée en ce qu'elle renferme, en association avec un excipient physiologiquement acceptable, la 1-éthyl-3-hydroxy-3-phénylpipéridine ou l'un de ses sels d'addition non-toxiques.

4. Utilisation d'une substance appartenant à l'ensemble comprenant la 1-éthyl-3-hydroxy-3-phénylpipéridine de formule I

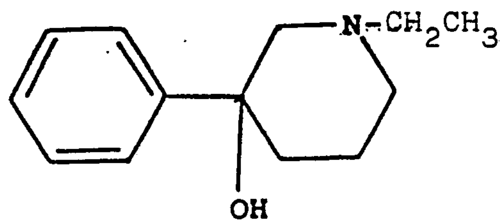

(I)

et ses sels d'addition non-toxiques, pour l'obtention d'un médicament anti-dépresseur du système nerveux central destiné à une utilisation en thérapeutique humaine vis-à-vis des dépressions et des états dépressifs.

5. Procédé de préparation de la 1-éthyl-3-hydroxy-3-phénylpipéridine de formule I selon la revendication 1 et de ses sels d'addition, caractérisé en ce que l'on fait réagir un halogénure de phénylmagnésium de formule II

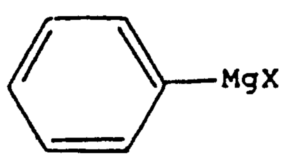

(II)

où X est un atome d'halogène, notamment de Cl, Br ou I, avec l'éthyl-3-pipéridinone de formule III

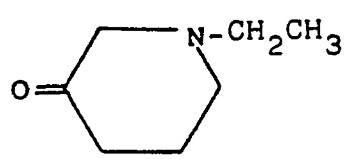

(III)

pendant au moins 0,25 h à une température comprise entre 0 et 25° C, sous atmosphère d'azote dans un solvant inerte notamment choisi parmi les éthers, le tétranydrofuranne et leurs mélanges.

6. Procédé de préparation de la 1-éthyl-3-hydroxy-3-phénylpipéridine de formule I selon la revendication 1 et de ses sels d'addition, caractérisé en ce que l'on fait réagir un halogénure d'éthyle de formule $YCH_2CH_3$, où Y est un atome d'halogène, avec la 3-hydroxy-3-phénylpipéridine de formule VI

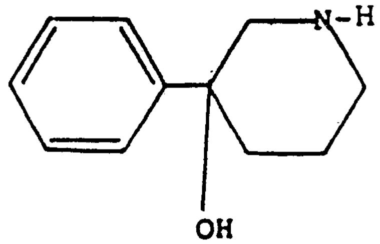

(VI)

à la température de reflux du milieu réactionnel.

**Revendications** pour l'état contractant: AT

1. Procédé de préparation de la 1-éthyl-3-hydroxy-3-phénylpipéridine et de ses sels d'addition, caractérisé en ce que l'on fait réagir un halogénure de phenylmagnesium de formule II

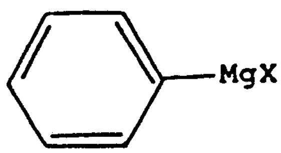

(II)

où X est un atome d'halogène, notamment de Cl, Br ou I, avec l'éthyl-3-pipéridinone de formule III

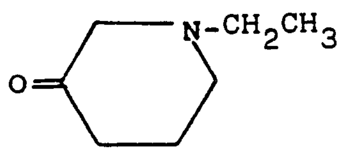

(III)

pendant au moins 0,25 h à une température comprise entre 0 et 25° C, sous atmosphère d'azote dans un solvant inerte notamment choisi parmi les éthers, le tétrahydrofuranne et leurs mélanges.

2. Procédé de préparation de la 1-éthyl-3-hydroxy-3-phénylpipéridine et de ses sels d'addition, caractérisé en ce que l'on fait réagir un halogénure d'éthyle de formule $YCH_2CH_3$ où Y est un atome d'halogène, avec la 3-hydroxy-3-phénylpipéridine de formule VI

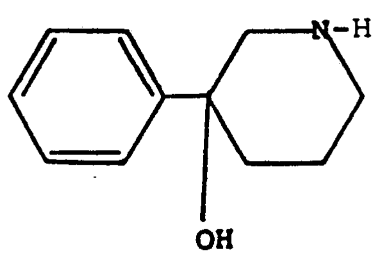

(VI)

à la température de reflux du milieu réactionnel.

**Patentansprüche** für die Vertragsstaaten: BE, CH, DE, FR, GB, IT, LI, LU, NL, SE

1. 3-Hydroxy-3-phenylpiperidinderivat, dadurch gekennzeichnet, daß es aus der Gruppe bestehend aus:
a) dem 1-Ethyl-3-hydroxy-3-phenylpiperidin der Formel I

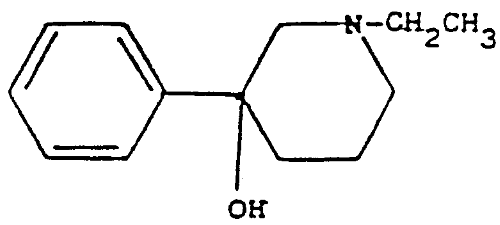

(I)

und

b) seinen Additionssalzen ausgewält ist.

2. 1-Ethyl-3-hydroxy-3-phenylpiperidinhydrochlorid.

3. Therapeutische Zusammensetzung, dadurch gekennzeichnet, daß sie zusammen mit physiologisch annehmbaren Hilfsstoffen 1-Ethyl-3-hydroxy-3-phenylpiperidin oder dessen nicht toxische Additionssalze enthält.

4. Verwendung eines Stoffes der Gruppe bestehend aus 1-Ethyl-3-hydroxy-3-phenylpiperidin der Formel I

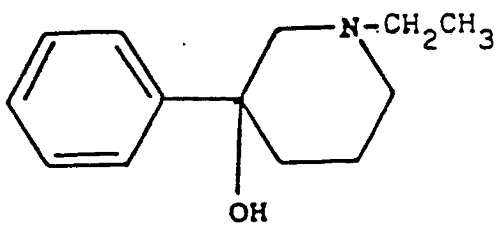

(I)

und dessen nicht toxische Additionssalze zur Herstellung eines auf das zentrale Nervensystem wirkenden antidepressiven Medikaments, welches zur Verwendung in der Humantherpie gegen Depressionn und Depressionszustände bestimmt ist.

5. Verfahren zur herstellung des 1-Ethyl-3-hydroxy-4-phenyl-piperidins der Formel I gemäß Anspruruch 1 und von dessen Additionssalzen, dadurch gekennzeichnet, daß man ein Phenylmagnesiumhalogenid der Formel II

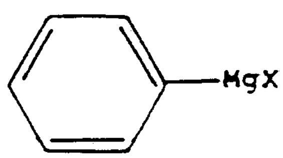

(II)

worin X ein Halogenatom, insbesondere Cl, Br oder I, ist, mit dem Ethyl-3-piperidinon der Formel III

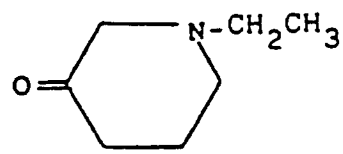

(III)

während wenigstens 0,25 bei einer Temperatur zwischen 0 und 25°C unter einer Stickstoffatomosphäre in einem inerten Lösungsmittel, insbesondere ausgewählt aus Ethern, Tetrahydrofuran und deren Gemischen, umsetzt.

6. Verfahren zur Herstellung des 1-Ethyl-3-phenylpiperidins gemäß Formel 1 und von dessen Additionssalzen, dadurch gekennzeichnet, daß man ein Ethylhalogenid der Formel $YCH_2CH_3$, worin Y ein Halogenatom ist, mit dem 3-Hydroxy-3-phenylpiperidin der Formel VI

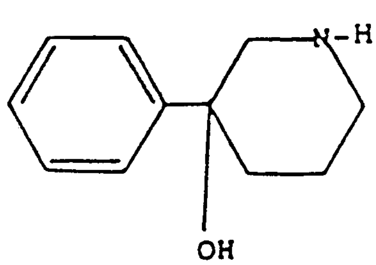

(VI)

bei der Rückflußtemperatur des Reaktionsgemisches umsetzt.

**Patentansprüche** für den Vertragsstaat: AT

1. Verfahren zur Herstellung von 1-Ethyl-3-hydroxy-4-phenyl-piperidin und von dessen Additionssalzen, dadurch gekennzeichnet, daß man ein Phenylmagnesiumhalogenid der Formel II

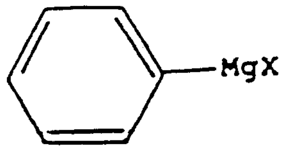

(II)

worin X ein Halogenatom, insbesondere Cl, Br oder I, ist, mit Ethyl-3-piperidinon der Formel III

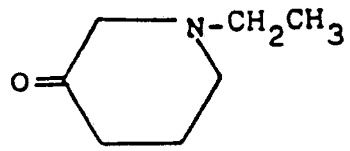

(III)

während wenigstens 0,25 h bei einer Temperatur zwischen 0 und 25°C unter einer Stickstoffatomosphäre in einem inerten Lösungsmittel, insbesondere ausgewählt aus Ethern, Tetrahydrofuran und deren Gemischen, umsetzt.

2. Verfahren zur Herstellung von 1-Ethyl-3-phenylpiperidin und von dessen Additionssalzen, dadurch gekennzeichnet, daß man ein Ethylhalogenid der Formel $YCH_2CH_3$, worin Y ein Halogenatom ist, mit dem 3-Hydroxy-3-phenylpiperidin der Formel VI

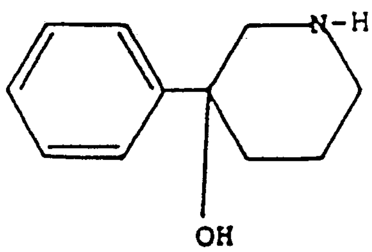

(VI)

bei der Rückflußtemperatur des Reaktionsgemisches umsetzt.

**Claims** for the contracting states: BE, CH, DE, FR, GB, IT, LI, LU, NL, SE

1. A 3-hydroxy-3-phenylpiperidine derivative which is selected from the group consisting of:
a) the 1-ethyl-3-hydroxy-3-phenylpiperidine compound of the formula I

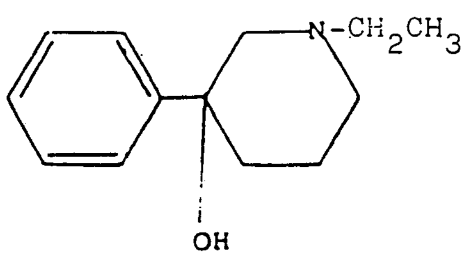

(I)

and,
b) addition salts thereof.

2. 1-Ethyl-3-hydroxy-3-phenylpiperidine hydrochloride.

3. A therapeutical composition which comprises, in association with a physiologically acceptable excipient, a pharmaceutically effective amount of 1-ethyl-3-hydroxy-3-phenylpiperidine or one of its non-toxic addition salts.

4. The use of a substance belonging to the group comprising the 1-etyl-3-hydroxy-3-phenylpiperidine of the formula I

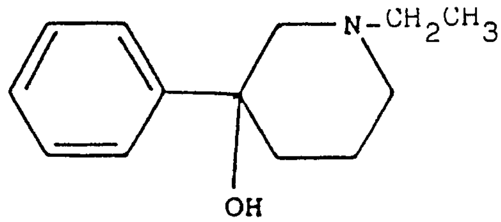

(I)

and non-toxic addition salts thereof for obtaining a CNS antidepressive medicament destined to be used in human therapy against depressions and depressive states.

5. A method for preparing the 1-ethyl-3-hydroxy-3-phenylpiperidine compound of the formula I according to claim 1 and its addition salts, which comprises reacting a phenylmagnesium halide of the formula II

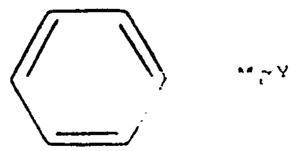

(II)

wherein X is Cl, Br or I, with an ethyl-3-piperidinone of the formula III

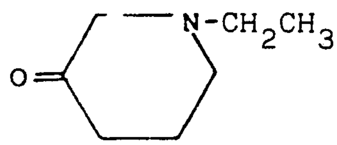

(III)

for at least 0.25 h, at a temperature comprised between 0 and 25°C, under a nitrogen atmosphere, in an inert solvent, in particular selected from ethers, tetrahydrofuran and mixtures thereof.

6. A method for preparing the 1-ethyl-3-hydroxy-3-phenylpiperidine compound of the formula I according to claim 1 and its addition salts, which comprises reacting an ethyl halide of the formula $YCH_2CH_3$ wherein Y is a halogen atom, with the 3-hydroxy-3-phenylpiperidine compound of the formula VI

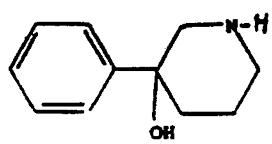

(VI)

at the reflux temperature of the reaction medium.

**Claims** for the contracting state: AT

1. A method for preparing the 1-ethyl-3-hydroxy-3-phenylpiperidine compound of the formula I

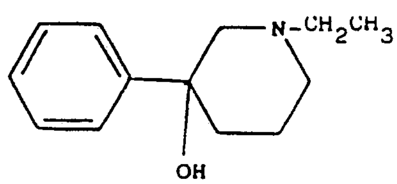

(I)

and its addition salts, which comprises reacting a phenylmagnesium halide of the formula II

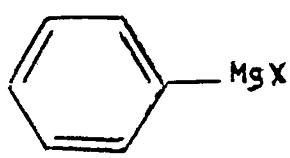

(II)

wherein X is Cl, Br or I, with an ethyl-3-piperidinone of the formula III

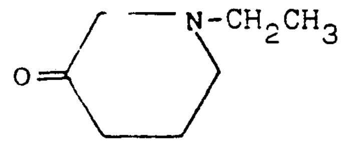

(III)

for at least 0.25 h, at a temperature comprised between 0 and 25°C, under a nitrogen atmosphere, in an inert solvent, in particular selected from ethers, tetrahydrofuran and mixtures thereof.

2. A method for preparing the 1-ethyl-3-hydroxy-3-phenylpiperidine compound and its addition salts, which comprises reacting an ethyl halide of the formula $YCH_2CH_3$, wherein Y is a halogen atom, with the 3-hydroxy-3-phenylpiperidine compound of the formula VI

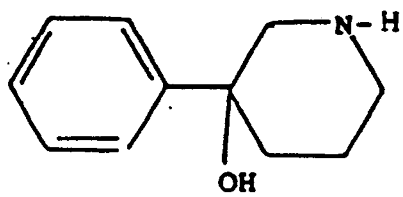

(VI)

at the reflux temperature of the reaction medium.